# EUROPÄISCHE PATENTSCHRIFT

(11) **EP 0 496 071 B1**
(45) Veröffentlichungstag und Bekanntmachung des Hinweises auf die Patenterteilung: **18.10.1995**
(21) Anmeldenummer: 91121248.8
(22) Anmeldetag: 11.12.1991
(51) Int. Cl.: A61F 5/30, A61F 13/06, A61F 13/10

(54) **Gelenkbandage**
Joint bandage
Bandage articulaire

(30) Priorität: 24.01.1991 DE 4101965
(43) Veröffentlichungstag der Anmeldung: 29.07.1992
(73) Patentinhaber: Beiersdorf Aktiengesellschaft, 20245 Hamburg (DE)
(72) Erfinder: Brandt, Dieter, W-4000 Düsseldorf (DE); Szlema, Ingeborg, W-4152 Kempen (DE); Wetz, Hans Henning, Dr.med., CH-8713 Uerikon (CH)
(74) Vertreter: Richter, Werdermann & Gerbaulet

(56) Entgegenhaltungen:
- EP-A- 0 126 256
- WO-A-85/04569
- US-A- 2 323 826
- US-A- 2 524 326
- US-A- 4 479 495

## Beschreibung

Die Erfindung betrifft eine Gelenkbandage aus elastischem Bandagenstoff, insbesondere in Schlauchform oder Kappenform, zur schnelleren Wiederherstellung der Funktion von verletzten Gelenken, wie Knie-, Schulter-, Ellenbogen- und/oder Handgelenken, mit mindestens einer im angelegten Zustand das Gelenk beaufschlagenden, als Druckpelotte ausgebildeten Einlage.

Das Bandagieren von Gelenken, z.B. nach Verletzungen oder bei degenerativen Erkrankungen,ist eine bekannte orthopädische Behandlungsmethode, wobei das Spektrum vom einfachen Wickeln mit mehr oder weniger elastischen Binden über Fertigbandagen in den verschiedensten Ausführungsformen bis zu aufwendig konstruierten Schienen und Braces reicht. Die dabei eingesetzten Bandagen sind meist schlauch- oder strumpfförmig, rund oder flach gestrickt und bei einigen Ausführungsformen durch abgeplattete Spiralfedern oder Kunststoffstäbe seitlich verstärkt.

Durch die EP-A-0 027 172 ist eine Bandage aus elastischem Bandagenstoff, insbesondere in Schlauchform, für die Abstützung bzw. Kompression von Knie-, Sprung-, Ellenbogen- und/oder Handgelenken, mit wenigstens einer im angelegten Zustand die Knochenvorsprünge des Gelenkes umgebenden, die benachbarten Gelenkweichteile beaufschlagenden Kompressionseinlage bekannt, bei der die Kompressionseinlage aus elastischem, jedoch inkompressiblem Silikonkautschuk oder einem Material mit gleichen Elastizitäts- und Kompressionseigenschaften besteht. Durch die Verwendung eines elastischen, jedoch inkompressiblen Silikonkautschuks als Material für die Kompressionseinlage wird bei angelegter Bandage eine nur auf die Oberfläche der Gelenkweichteile wirkende Massage ohne Tiefenwirkung erreicht, da ein Silikonkautschuk, der elastische und inkompressible Eigenschaften aufweisen soll, eine geringe Rückprallelastizität aufweist, so daß die für eine Massagewirkung erforderliche Materialverschiebung bei Druckbeaufschlagung nicht in dem gewünschten Maße eintritt und eine Massage mit einer Tiefenwirkung nicht erreichbar ist. Hinzu kommt noch, daß es für eine gute Durchblutung der Gelenkweichteile erforderlich ist, eine ausreichende Wechselbelastung zu erzeugen, aufgrund der das Blut aus den unter der Kompressionseinlage befindlichen Gelenkweichteilen herausgedrückt wird und bei Entlastung wieder zurückströmt.Mit einem elastischen, inkompressiblen Silikonkautschuk ist aber eine derartige Wechselbelastung nicht erreichbar, da ausgeübte lokale Druckkräfte auf die der Druckkomponente zugeordnete Abstützfläche nicht erzeugbar sind. Das ist darauf zurückzuführen, daß der bei dieser Bandage als Kompressionseinlage eingesetzte Silikonkautschuk bei Druckeinwirkung nicht komprimiert wird, sondern es tritt lediglich eine Masseverschiebung in dem Sinne ein, daß die aus den vom Druck beanspruchten Bereichen verdrängten Masseanteile seitlich auswandern, und zwar in benachbarte Bereiche, die nicht druckbeansprucht sind, so daß in diesen benachbarten Bereichen eine Massevermehrung eintritt, die in Verbindung mit der angelegten Bandage auf die entsprechenden Bereiche des Gewebes eine Massagewirkung ausübt. Eine voranstehend erwähnte Wechselbelastung wird jedoch nicht erreicht; erhalten wird jedoch eine gute Oberflächenmassage, aber keine Massage mit Tiefenwirkung und somit keine Durchblutungsverbesserung.

Eine Epicondylitis-Bandage ist durch die EP-A-0 250 409 bekannt. Eine derartige Epicondylitis-Bandage besteht im wesentlichen aus einem Schlauchabschnitt aus elastischem Material, wobei die Zugspannung in Umfangsrichtung über einen im wesentlichen in Umfangsrichtung verlaufenden Spannriemen mit Verschluß veränderbar ist, wobei sich der erwähnte Schlauchabschnitt im wesentlichen gleichmäßig zu beiden Seiten um das Ellenbogengelenk erstreckt. Bei dem elastischen Material des Schlauchabschnittes handelt es sich um ein Gestrick mit wärmedämmender Wirkung. An den den Epicondylen entsprechenden Stellen sind im wesentlichen plattenförmige, hartelastische Einlagen vorhanden, wobei der Spannriemen zwischen den erwähnten Einlagen diese zumindest teilweise übergreifend angeordnet ist. Mit einer derart ausgebildeten Epicondylitis-Bandage wird die Druckwirkung durch einen den Arm umgreifenden Riemen mit Hilfe von Plättchen erzeugt, die den Positionen der Epicondylen zugeordnet sind. Riemen und Plättchen sind dabei fest mit einer elastischen Armbandage verbunden. Die Armbandage umschließt dabei einen Teil des Unterarms,des Ellbogens und einen Teil des Ober-arms und ist im höchsten Maße rutschfest und soll die medizinisch richtige Lage von Spannriemen mit Plättchen auch bei nur geringer Spannung des Spannriemens garantieren. Eine zusätzliche Wärmewirkung der Bandage soll insbesondere in den Fällen erreicht werden, in denen eine wärmeisolierende, textile oder andere Lage zusätzlich vorgesehen ist,wobei jedoch ein zusätzliches wärmeisolierendes Fadenmaterial in das Gewebe oder Gestrick aufgenommen sein kann.

Eine Bandage zur Behandlung von Epicondylitis beschreibt ferner die DE-U-84 10 987.4. Diese Bandage, die zur Behandlung von Epicondylitis durch Ausüben eines Druckes auf die Streckmuskulatur des Unterarmes dient, besteht aus einem Gurt zur Entlastung der an die Epicondylen angreifenden Muskeln mit an seinen Enden angeordnetem Halteverschluß zur Bildung eines einstellbaren Ringelementes, wobei der Gurt mindestens eine Druckplatte bzw. zwei im Abstand angeordnete Druckplatten trägt und zur Ausrichtung bzw. zur gegenüberliegenden Anordnung der Druckplatten am Unterarm die Druckplatten verschiebbar und einstellbar am Gurt angeordnet sind. Mit einer derart ausgebildeten Bandage soll es möglich sein, unter Berücksichtigung des Tragekomforts eine funktionsfähige, individuell anpassungsfähige Anordnung auszubilden, die die Einstellung der Druckplatten in die erforderliche Position ermöglicht.

Durch die DE-A-34 12 772 ist eine Knie-Orthese bekannt, die aus einem strumpfförmigen, dehnbaren Gestrick mit im Bereich der Kniescheibe angeordneter, ringförmiger Pelotte aus elastischem, inkompressiblem Werkstoff besteht. Um die Fläche, mit der die Pelotte die Körperoberfläche berührt, zu erhöhen und um durch diese Maßanahme die Pelotte selbst und damit auch die gesamte Orthese am Knie rutschfester zu positionieren, weist die ringförmige Pelotte seitliche Fortsätze auf,die sich bis in die Bereiche seitlich des Kniegelenkes erstrecken. In die seitlichen Fortsätze sind Verstärkungen eingearbeitet, die aus elastischem, aber festem Werkstoff bestehen und in die Pelotte eingegossen oder einvulkanisiert sind. Dadurch, daß bei dieser Knie-Orthese von einer ringförmigen Pelotte ausgegangen wird, ist diese Orthese ausschließlich am Kniegelenk einsetzbar, so daß die ringförmige Pelotte im Bereich der Kniescheibe zu liegen kommt. Die Pelotte umschließt mit ihrem ,die mittige Durchbrechung begrenzenden Rand den seitlichen Bereich der Kniescheibe. Bei anderen Gelenken, wie z.B. beim Sprung-, Schulter- und/oder Handgelenk ist diese Orthese nicht einsetzbar. Die Pelotte selbst besteht aus elastischem, jedoch inkompressiblem Silikon-Kautschuk, wohingegen die Verstärkungen aus elastischem, aber festem Werkstoff gefertigt sind. Daß die Pelotte und ihre Verstärkungen in den seitlichen Pelottenfortsätzen unterschiedliche Härtegrade aufweisen, ist ebenso nicht vorgesehen, wie eine Differenz der Shore-Härten zwischen der Pelotte und den Verstärkungen.

Bei diesen bekannten Bandagen werden Kompressionseinlagen und Druckplatten verwendet, die gelenkseitig zugewandt keine speziell ausgebildete Oberflächenstruktur aufweisen, sondern flach ausgebildet sind,wobei im ersteren Fall der Kompressionseinlage ein Druck auf die Gelenkweichteile zur Erzeugung eines mechanischen, ein schnelles Abschwellen der Ergüsse von Gelenkverletzungen bewirkenden Reizes durch Erzielung eines, eine intensive Massage der Gelenkweichteile bewirkenden Rolleffektes aufgrund des verwendeten elastischen, jedoch inkompressiblen Silikonkautschuks,ausgeübt wird, wohingegen die verwendeten Druckplatten lediglich einen erhöhten Andruck der Sehnenfasern in ihren Ansatzpunkten, nämlich den Epicondylen, bewirken, jedoch wird in beiden Fällen keine gezielte Friktionsmassage auf spezielle Schmerzpunkte des Gelenkes ausgeübt, d.h. es ist keine Massageform gegeben, bei der die Sehnenansätze quer zum Ansatz behandelt werden. Die Kombination einer Friktionsmassage mit einer Gelenkweichteilmassage ist mit keiner der bekannten Bandagen erreichbar.

Der Erfindung liegt die Aufgabe zugrunde, eine Gelenkbandage der eingangs genannten Art zu schaffen, die über eine gezielte, streng lokal angesetzte Querfriktionsmassage schonend eine aktive Übungsbehandlung unterstützt und damit eine schnellere Wiederherstellung der Gelenkfunktionen von verletzten Gelenken ermöglicht, sowie über den Effekt der Schmerzlinderung hinaus eine heilende Wirkung ausübt.

Diese Aufgabe wird durch die im Patentanspruch 1 gekennzeichneten Merkmale gelöst.

Eine derart erfindungsgemäß ausgebildete Bandage unterstützt schonend die aktive Übungsbehandlung und ermöglicht damit eine schnellere Wiederherstellung der Gelenkfunktionen. Neben dem Effekt einer Schmerzlinderung wird eine heilende Wirkung erzielt. Die mit der Bandage erreichte Wirkung basiert auf den Faktoren Entlastung,Kompression und Friktionsmassage, wobei die Bandage selbst das Gelenk führt, polstert und begrenzt stabilisiert. Es wird ein Kompressionsdruck auf die Gelenkweichteile und Sehnenansätze erzeugt und bei Bewegung eine intermittierende Massage auf die beteiligten Muskeln ausgeübt.Aufgrund des in der Druckpelotte der Bandage angeordneten harten Friktionskerns ist neben einer Massage der Gelenkweichteile durch das Pelottenmaterial eine gezielte Friktionsmassage auf spezielle Schmerzpunkte möglich. Bei dieser Massageform werden die Sehnenansätze quer zum Ansatz behandelt, wobei die Querfriktion streng lokal angesetzt wird. Diese Friktionsmassage wirkt in akuten Fällen einer Adhäsionsbildung mit angrenzenden Strukturen entgegen und löst in subakuten oder chronischen Fällen bestehende Adhäsionen. Gleichzeitig beseitigt die Friktionsmassage durch die Freisetzung von Histamin und Serotonin aus zerstörten Mastzellen lokale Entzündungsreaktionen biochemisch und führt somit zu einer Schmerzfreiheit. Die ideale orthometrische Form ist für jedes Gelenk verschieden und wird der anatomischen Form des jeweiligen Gelenkes angepaßt, wobei die Bandagen für Knie-, Sprung-, Schulter-,Ellenbogen- und/oder Handgelenke einen gleichen vorzugsweisen Grundaufbau aufweisen:
- dreidimensional anatomische Formstrickung in funktionaler Beugestellung des Gelenkes,
- Kompressionsdruck [15 mm Hg] 2 x 10³ Pa,
- Zweizugelastik und damit ausgeglichene Druckverteilung in beide Richtungen,
- besonders ausgeführte Abschlüsse der Bandagenränder mit Druckverminderungsrand, so daß keine Stauungsbeschwerden auftreten,soweit es sich um schlauchförmige Bandangen handelt,
- viskoelastische Profileinlagen aus Silikon als Aktivelemente; Knochenvorsprünge und Sehnenansätze bestimmen die Form der Pelotte,
- Zwei-Komponentenpelotte mit einem festen, harten Friktionskern zur gezielten Friktionsmassage der kritischen Punkte (Sehnenansätze),
- Einsätze aus hochelastischem Relief-Gestrick, in deren Bereich das Material nach dem Faltenbalg-Prinzip wellenförmig gerafft wird. Die Wellen erlauben dabei eine hohe Beweglichkeit, nehmen in der Gelenkbeugung das überschüssige Material auf und verhindern eine Faltenbildung,
- hautfreundliches Gewebe aus z.B. vernetztem Polyurethan (Handelsname ELASTHAN),Elastodienfasern und Polyamid mit hohem Baumwollanteil, wobei auch Elastofasern eingesetzt werden können, die aufgrund ihres chemischen Aufbaus extrem hoch verformbar sind und die Eigenschaft aufweisen, nach Aufheben der Verformungskräfte im wesentlichen augenblicklich und fast vollständig in den ursprünglichen Zustand zurückzukehren, d.h. es sind solche hochelastischen Fasern, die eine hohe elastische Dehnung besitzen. Diese hochelastischen Fasern können aus Gummifäden, aus Kautschuk und anderen synthetischen Elastofasern bestehen, die nicht auf der Basis von Polyurethan hergestellt sind. Bei den Elastodienfasern handelt es sich um Fasern aus natürlichem Polyisopren (Gummi) oder synthetischem Polyisopren oder aus solchen Polymeren, die durch Polymerisation eines oder mehrerer Diene, evtl. unter Zusatz eines oder mehrerer Vinylmonomere,entstehen.

Vorteilhafte Ausgestaltungen der Erfindung sind in den Unteransprüchen gekennzeichnet.

Ausführungsbeispiele der Erfindung werden nachstehend anhand der Zeichnung näher erläutert. Es zeigen
Fig. 1 in einer Ansicht von oben eine Gelenkbandage mit an dieser angeordneten Druckpelotte,
Fig. 2 in einer Ansicht von oben die Druckpelotte,
Fig. 3 einen senkrechten Längsschnitt gemäß Linie III-III in Fig. 2,
Fig. 4 in einer vergrößerten Seitenansicht einen Friktionskern.
Fig. 5 in einer Ansicht von oben die Druckpelotte mit in den bogenförmigen Schmalseitenbereichen des Pelottenformkörpers dargestellten Grundkreisen und
Fig. 6 in einem senkrechten Längsschnitt eine weitere Ausführungsform einer Druckpelotte mit einem in deren Material eingearbeiteten Friktionskern.

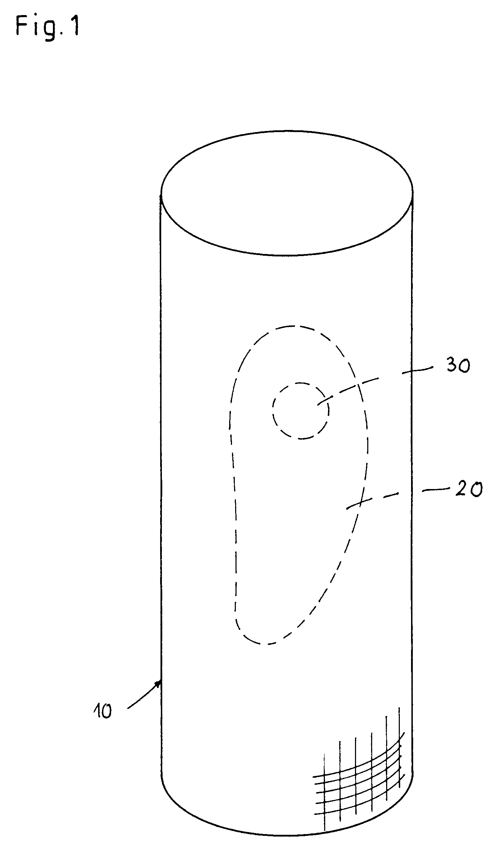

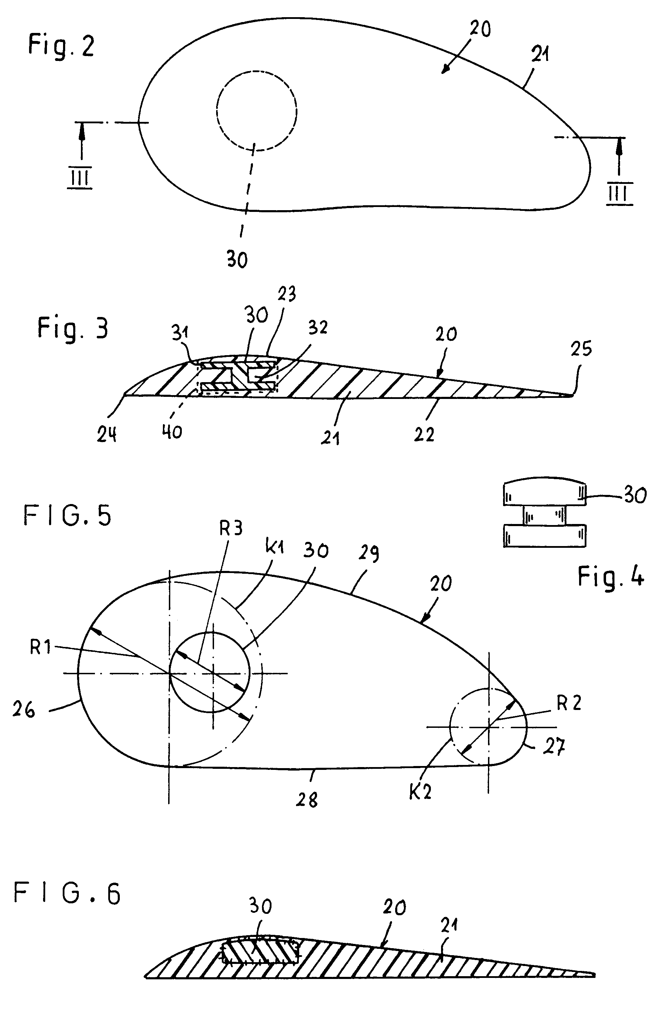

Die in Fig. 2 und 3 dargestellte und mit 20 bezeichnete Druckpelotte findet Verwendung als Einlage in einer vorzugsweise schlauchförmig oder kappenförmig ausgebildeten Bandage 10 aus einem elastischen Bandagenstoff, z.B. einem elastischen Gewebe, Gewirke oder Gestricke (Fig.1). Eine derartige Gelenkbandage 10 ist aufziehbar auf ein Knie-, Sprung-, Schulter-, Ellenbogen- und/oder Handgelenk, wobei im angelegten Zustand die Druckpelotte 20 das Gelenk derart beaufschlagt, daß ein Kompressionsdruck auf die Gelenkweichteile und Sehnenansätze und eine gezielte Friktionsmassage auf spezielle Schmerzpunkte ausgeübt wird. Für die Befestigung der Druckpelotte 20 an der Bandage 10 ist diese im Befestigungsbereich bevorzugterweise doppellagig ausgebildet, wobei dann in dem durch diese Doppellagigkeit ausgebildeten Zwischenraum die Druckpelotte 20 angeordnet ist, die die in Fig. 2 und 3 dargestellte Form aufweist. Jedoch auch andere bekannte Befestigungsformen für die Druckpelotte, z.B. wie Schweiß-, Klebe- oder Nähverbindungen, sind möglich.

Die Druckpelotte 20 ist als Formkörper 21 ausgebildet und weist eine durch die Knochenvorsprünge und Sehnenansätze des Gelenks vorgegebene Form auf. Der Formkörper 21 der Druckpelotte 20 besteht aus einem weich-elastischen Material.

In dem Material des Formkörpers 21 ist ein fester Friktionskern 30 fixiert, der aus einem harten Material, wie z.B. einem inkompressiblen Polyamid, Polyurethan, Silikonkautschuk oder einem Material mit gleichen Elastizitätseigenschaften oder einem unelastischen Material besteht. Dieser Friktionskern 30 ist scheibenförmig oder balkenförmig mit einem kreisförmigen oder eine andere geometrische Form aufweisenden Querschnitt ausgebildet und weist im Bereich seiner umlaufenden Wandfläche 31 eine Einziehung in Form einer Nut 32, rillenförmigen Ausnehmungen, Hinterschneidungen, Verzahnungen od.dgl. auf, die zur Aufnahme des Formkörpermaterials dient, damit der Friktionskern 30 in dem Formkörper 21 der Druckpelotte 20 in seiner Lage fixiert ist. Bevorzugterweise ist die Anordnung des Friktionskern 30 so getroffen, daß dieser dicht unterhalb der Oberfläche des Formkörpers 21 zu liegen kommt, um so einen hohen Druck auf die Gelenk ausüben zu können (Fig.4).

Der Formkörper 21 besteht aus einem weichen oder weich-elastischen Material. Dagegen besteht der Friktionskern 30 aus einem harten oder inkompressiblen Material; er ist in dem Material des Formkörpers 21 in seiner Lage fixiert. Der Friktionskern 30 weist gegenüber der Härte des Formkörpers 21 eine größere Härte auf. Die Differenz zwischen der Härte des Formkörpers 21 und der Härte des Friktionskerns 30 beträgt mindestens 10 Shore A, vorzugsweise 20 Shore A.

Die Härte des Materials, aus dem der Formkörper 21 besteht, liegt unterhalb 50 Shore A, wohingegen die Härte des Materials, aus dem der Friktionskern 30 gefertigt ist, oberhalb 50 Shore A liegt, worauf nachstehend noch näher eingegangen wird.

Die Druckpelotte 20 weist bevorzugterweise die in Fig. 2 und 3 dargestellte Form auf. Hiernach ist der Formkörper 21 mit einer flachen Grundfläche 22 und einer der Grundfläche abgewandten Außenwölbung 23 versehen, die sich von einem Ende 24 zum anderen Ende 25 des Formkörpers 21 konisch verjüngt. Der Friktionskern 30 ist in dem Formkörper 21 vorzugsweise in demjenigen Bereich der Außenwölbung 23 angeordnet, der den größten Querschnitt aufweist (Fig.3).

Der Formkörper 21 selbst weist eine etwa rechteckige Form mit konisch sich zu dem Formkörperende 25 hin verjüngenden Längsseiten 28,29, von denen die Längsseite 29 bogenförmig und die Längsseite 28 fast geradlinig, vorzugsweise mit einer geringfügigen Einziehung verlaufen, und mit bogenförmig ausgebildeten Schmalseiten 26,27 auf, wobei den beiden bogenförmigen Schmalseiten unterschiedliche Kreisdurchmesser R1,R2 zugrunde liegen. Der Kreisdurchmesser R1 des der bogenförmigen Schmalseite 26 zugrunde liegenden Kreises K1 weist bei dem in Fig. 5 gezeigten Ausführungsbeispiel etwa 4 cm und der Kreisdurchmesser R2 des der bogenförmigen Schmalseite 27 zugrunde liegenden Kreises K2 etwa 2 cm auf, wobei der Formkörper 21 eine Länge von etwa 11 cm und eine Höhe der Außenwölbung 23 von etwa 1 cm im Bereich der Schmalseite 26 beträgt. Eine Druckpelotte 20 mit diesen Abmessungen ist beispielsweise in einer Ellenbogen-Bandage verwendbar. Abweichungen von diesen Maßen sind möglich.

Der Formkörper 21 besteht aus einem weichen oder weich-elastischen Material, wie Filz, Moosgummi, Neopren, Gummi oder aus viskoelastischen Silikonkautschuk oder aus einem elastischen, kompressiblen, durch Druck verformbaren Silikonkautschuk z.B. mit einer Härte von 40 Shore A, einem Silikonschaum mit einer Härte von 9 bis 13 Shore A oder einem kompressiblen, durch Druck verformbaren und ohne Sprungelastizität in seine Form sich rückbildenden Silikonkautschuk vom Typ eines nach dem Verfahren der Polyaddition vulkanisierenden Kaltkautschuks, der neben einer hohen Flexibilität eine unter 4 Shore A liegende Härte aufweist, wobei jedoch auch Silikonkautschuke eingesetzt werden können, deren Härte oberhalb von 4 Shore A liegt. Derartige viskoelastische Silikonkautschuke oder Materialien mit gleichen Elastizitätseigenschaften besitzen die Eigenschaft, bei angelegter Bandage mit einer derartigen Druckpelotte aufgrund der gleitenden Bewegung, ausgelöst durch Masseverschiebung, bei Druckanwendung oder bei Bewegungsabläufen im Auflagebereich massierend zu wirken. Für die Herstellung des Formkörpers 21 sollte ein Material gewählt werden, welches viskoelastisch ist und aufgrund seiner elastischen Eigenschaften eine Massage bewirkt.

Der Friktionskern 30 der Druckpelotte 20 besteht dagegen aus einem harten oder inkompressiblen Kunststoff mit z.B. einer über 50 Shore A liegenden Härte und weist gegenüber dem Formkörper 21 eine weitaus größere Härte auf, damit in der Bewegung eine gezielte Friktionsmassage auf spezielle Schmerzpunkte der Gelenke erreicht wird. Als Material für den Friktionskern kommt natürlicher oder künstlicher Kautschuk oder Hartgummi infrage. So kann u.a. ein Chloropren-Polymerisat ( Handelsname NEOPREN) mit einer Härte von 50 Shore A, ein gummielastisches, vernetztes Polyurethan (Handelsname VULKOLLAN) mit einer Härte von 65 bis 90 Shore A , ein Silikonkautschuk mit einer Härte von 60 Shore A, ein Ethylen-Propylen-Dien-Kautschuk (EPDM) mit einer Härte von 80 Shore A oder ein Copolymerisat mit Acrylnitril (Handelsname PERBUNAN) mit einer Härte von 70 Shore A, ferner ein Polyamid verwendet werden. Jedoch auch andere Kunststoffe oder Naturstoffabkömmlinge können zur Herstellung des Kerns 30 herangezogen werden. Wesentlich ist, daß der Kern 30 eine ausreichende Härte aufweist, um die gezielte Friktionsmassage auf spezielle Schmerzpunkte ausüben zu können. Der Friktionskern 30 kann auch aus Metall oder Holz bestehen.

Die Anordnung der Druckpelotte 20 in der Bandage ist derart, daß die Außenwölbung 23 der Druckpelotte 20 dem Gelenk zugekehrt ist. Je nach Gelenkart können in der Bandage 10 auch mehr als eine Druckpelotte angeordnet sein. Auch kann die Druckpelotte mehrere einzelne Friktionskerne 30 aufweisen, um mehrere Schmerzpunkte gleichzeitig zu beaufschlagen.

Der Friktionskern 30, der eine Knopfform oder jede andere geometrische Form aufweist, ist in die Druckpelotte 20 fest eingezogen und schließt an der Oberfläche der Druckpelotte kugelförmig ab. Zur Lagensicherung, d.h. zur Sicherung gegen ein Verschieben oder Wandern, des Kerns 30 in der Druckpelotte 20 ist der Kern mit einer Oberflächenprofilierung, mit Hinterschneidungen oder mit Verzahnungen versehen, in die das Pelottenmaterial eingreift. Erforderlich ist, daß der Friktionskern 30 aus einem Material besteht, das gegenüber dem Material der Druckpelotte etwas Härter ist. So besteht auch die Möglichkeit, für die Druckpelotte ein Material mit einer Härte von 4 Shore A und für den Friktionskern 30 ein Material mit einer Härte von z.B. 15 Shore A zu verwenden. Bei angelegter Bandage liegt der Friktionskern 30 auf dem Sehnenansatz, wohingegen die Druckpelotte 20 selbst auf dem Muskel und/oder den Gelenkweichteilen aufliegt. Die Druckpelotte kann auch auf beiden Seiten der Bandage angeordnet sein.

Nach einer weiteren Ausführungsform der Erfindung ist der Friktionskern 30 in der Druckpelotte 20 auswechselbar angeordnet. Hierzu ist der Formkörper 21 mit einer Ausnehmung 40 von etwa der Größe des Friktionskerns 30 versehen, in die der Friktionskern 30 vermittels eines leichten Druckes eingedrückt wird (Fig.3). Die die Ausnehmung in der Druckpelotte 20 begrenzende Innenwandfläche weist eine Profilgebung auf, die einen Eingriff in das Profil der umlaufenden Wandfläche des Friktionskerns 30 ermöglicht und da das Pelottenmaterial elastisch ist, der Friktionskern 30 jedoch gegenüber dem Druckpelottenmaterial eine größere Härte aufweist, läßt sich der Friktionskern 30 in die Ausnehmung pressen, wobei während des Preßvorganges das Profil der Innenwandfläche so zusammengepreßt wird, daß der Kern 30 gänzlich in die Ausnehmung 40 gleiten kann und aufgrund des Rückstellvermögens des Materials der Druckpelotte 20 wird das Pelottenmaterial in das Randprofil des Friktionskern 30 gepreßt, so daß dieser fest in der Druckpelotte gehalten wird. Durch entsprechende Verformung der Druckpelotte durch eine starke äußere Druckanwendung kann der Kern 30 aus der Druckpelotte herausgedrückt werden. Dadurch ist die Möglichkeit gegeben, Friktionskerne unterschiedlicher Härte verwenden zu können. In dem Fall, in dem Druckpelotten 20 mit auswechselbarem Friktionskern 30 verwendet werden, ist die Druckpelotte an der Bandage derart befestigt, daß ein Abnehmen der Druckpelotte möglich ist.

Der Friktionskern 30 kann als Formkörper ausgebildet sein; er ist dann in dem Material des Formkörpers 21 angeordnet. Nach einer weiteren Ausführungsform der Erfindung ist das Material des aus einem Kunststoff, z.B. Silikonkautschuk bestehenden Friktionskerns 30 mit dem Material des Formkörpers 21 verschmolzen und mit dem Formkörper 21 unlösbar verbunden (Fig.6). Der Friktionskern 30 kann auch bei der Herstellung des Formkörpers 21 durch Materialaushärtung eines Abschnittes,der den späteren Friktionskern bilden soll und daher eine größere Härte gegenüber dem weichen Material des Formkörpers 21 aufweist,des Formkörpers 21 erhalten werden. In beiden Fällen sollte als Material bevorzugterweise Silikonkautschuk verwendet werden.

Des weiteren kann der Formkörper 21 auch beutelförmig ausgebildet sein. Dieser Beutel besteht aus weich-elastischen Kunststoffen. Der Innenraum des Beutels ist mit einem gasförmigen Medium, wie z.B. Luft,oder einem flüssigen Medium, wie z.B. einem zähflüssigen Silikonöl, Wasser od.dgl. gefüllt. Der Friktionskern 30 ist dann an der Innenwandfläche des Beutels in seiner Lage fixiert.

Neben der Ausbildung der Druckpelotte 20 mit einem oder mehreren Friktionskernen 30 kann die Bandage selbst auch an zwei einander zugekehrten Seiten Druckpelotten 20 mit Friktionskernen 30 tragen.

## Patentansprüche

1. Gelenkbandage aus elastischem Bandagenstoff, insbesondein Schlauchform oder Kappenform, zur schnelleren Wiederherstellung der Funktion von verletzten Gelenken, wie Knie-, Sprung, Schulter-, Ellenbogen- und/oder Handgelenken, mit mindestens einer im angelegten Zustand das Gelenk beaufschlagenden, als Druckpelotte ausgebildeten Einlage, dadurch gekennzeichnet, daß die Druckpelotte (20) eine durch die Knochenvorsprünge und Sehnenansätze des Gelenkes bestimmte Form aufweist und als Formkörper (21) aus einem weichen oder weich-elastischen Material ausgebildet ist, in dem mindestens ein Friktionskern (30) aus einem harten oder inkompressiblen Material angeordnet und in dem Material des Formkörpers (21) im Bereich der Ansätze der erkrankten Sehnen so fixiert ist, daß bei einer Bewegung eine Massage quer zum Faserverlauf dieser Sehnen erfolgt.

2. Gelenkbandage nach Anspruch 1, dadurch gekennzeichnet, daß der Friktionskern (30) eine gegenüber der Härte des Materials des Formkörpers (21) größere Härte aufweist.

3. Gelenkbandage nach einem der Ansprüche 1 und 2, dadurch gekennzeichnet, daß die Differenz zwischen der Härte des Formkörpers (21) und der Härte des Friktionskerns (30) mindestens 10 Shore A, vorzugsweise 20 Shore A, beträgt.

4. Gelenkbandage nach einem der Ansprüche 1 bis 3, dadurch gekennzeichnet, daß das Material des Formkörpers (21) eine unterhalb 50 Shore A liegende Härte und das Material des Friktionskerns (30) eine oberhalb 50 Shore A liegende Härte aufweist.

5. Gelenkbandage nach einem der Ansprüche 1 bis 4, dadurch gekennzeichnet, daß der Friktionskern (30) scheibenförmig oder balkenförmig mit einem kreisförmigen Querschnitt ausgebildet ist oder der Friktionskern (30) eine andere geometrische Form aufweist.

6. Gelenkbandage nach einem der Ansprüche 1 bis 5, dadurch gekennzeichnet, daß der scheibenförmige Friktionskern (30) zur Lagenfixierung in dem Formkörper (21) der Druckpelotte (20) im Bereich seiner umlaufenden Wandfläche (31) eine rillenförmige Ausnehmung, Einziehung, wie Nut, Hinterschneidungen, Verzahnung od. dgl. (32), zur Aufnahme von Formkörpermaterial aufweist.

7. Gelenkbandage nach einem der Ansprüche 1 bis 4, dadurch gekennzeichnet, daß das Material des Friktionskerns (30) mit dem Material des Formkörpers (21) verschmolzen und mit dem Formkörpermaterial unlösbar verbunden ist, wobei der Friktionskern (30) und der Formkörper (21) aus Kunststoffen, insbesondere Silikonkautschuken od. dgl. besteht.

8. Gelenkbandage nach einem der Ansprüche 1 bis 4, dadurch gekennzeichnet, daß der Friktionskern (30) bei der Herstellung des Formkörpers (21) durch Materialaushärtung eines Abschnittes des Formkörpers (21) erhalten wird, wobei der Friktionskern (30) und der Formkörper (21) aus Kunststoffen, insbesondere Silikonkautschuken od. dgl. unterschiedlicher Härtegrade besteht.

9. Gelenkbandage nach einem der Ansprüche 1 bis 8, dadurch gekennzeichnet, daß der Formkörper (21) eine flache Grundfläche (22) und eine dem Gelenk zugekehrte, der Grundfläche abgewandte Außenwölbung (23) aufweist.

10. Gelenkbandage nach Anspruch 9, dadurch gekennzeichnet, daß die Außenwölbung (23) des Formkörpers (21) sich von einem Ende (24) zum anderen Ende (25) konisch verjüngt und daß der Friktionskern (30) in dem Bereich der Außenwölbung (23) angeordnet ist, der den größten Querschnitt aufweist.

11. Gelenkbandage nach einem der Ansprüche 1 bis 10, dadurch gekennzeichnet, daß der Formkörper (21) eine etwa rechteckige Form mit konisch sich zu einem Formkörperende (25) verjüngenden Längsseiten (28, 29) und mit bogenförmig ausgebildeten Schmalseiten (26, 27) aufweist, wobei den bogenförmigen Schmalseiten (26, 27) unterschiedliche Kreisdurchmesser (R1, R2) zugrunde liegen.

12. Gelenkbandage nach Anspruch 11, dadurch gekennzeichnet, daß der Kreisdurchmesser (R1) des der bogenförmigen Schmalseite (26) zugrunde liegenden Kreises (K1) etwa 4 cm und der Kreisdurchmesser (R2) des der bogenförmigen Schmalseite (27) zugrunde liegenden Kreises (K2) etwa 2 cm bei einer Länge des Formkörpers (21) von etwa 11 cm und einer Höhe der Außenwölbung (23) von etwa 1 cm im Bereich der Schmalseite (26) beträgt.

13. Gelenkbandage nach einem der Ansprüche 1 bis 12, dadurch gekennzeichnet, daß der Formkörper (21) aus Filz, Moosgummi, Neopren, Gummi, einem viskoelastischen Silikonkautschuk oder aus einem elastischen, kompressiblen, durch Druck verformbaren Silikonkautschuk oder einem Material mit gleichen Elastizitätseigenschaften, wie Naturkautschuk, Silikonschaum od. dgl. besteht.

14. Gelenkbandage nach einem der Ansprüche 1 bis 12, dadurch gekennzeichnet, daß der Formkörper (21) beutelförmig ausgebildet ist und eine Füllung aus einem gasförmigen oder flüssen Medium aufweist, wobei der Friktionskern (30) an der Innenwandfläche des Beutels in seiner Lage fixiert ist.

15. Gelenkbandage nach einem der Ansprüche 1 bis 14, dadurch gekennzeichnet, daß der Friktionskern (30) aus einem inkompressiblen Kunststoff, wie einem natürlichen oder künstlichen Kautschuk, Hartgummi, Chloropren-Polymerisat, einem gummielastischen vernetzten Polyurethan, Polyamid, Metall, Holz od. dgl. besteht.

16. Gelenkbandage nach einem der Ansprüche 1 bis 15, dadurch gekennzeichnet, daß die Bandage (10) an zwei einander gegenüberliegenden Seiten Druckpelotten (20) mit Friktionskernen (30) trägt.

17. Gelenkbandage nach einem der Ansprüche 1 bis 16, dadurch gekennzeichnet, daß in dem Formkörper (21) der Druckpelotte (20) eine den Friktionskern (30) aufnehmende Ausnehmung (40) ausgebildet ist.

18. Gelenkbandage nach Anspruch 17, dadurch gekennzeichnet, daß der Friktionskern (30) in der Ausnehmung (40) lösbar vermittels Preß- oder Klemmsitz gehalten ist.

19. Gelenkbandage nach einem der Ansprüche 1 bis 18, dadurch gekennzeichnet, daß die obere Wandfläche des Friktionskerns (30) bogenförmig, halbkreisförmig oder flach mit abgerundeten Ecken ausgebildet ist.

## Claims

1. Joint bandage of elastic bandage cloth, more particularly in tubular or cap-like configuration, for the faster restoration of the function of injured joints, such as knee joint, ankle, shoulder joint, elbow joint and/or wrist, with at least one insert constructed in the form of a pressure pad acting upon the joint in the applied state,
characterized in that
the pressure pad (20) possesses a shape determined by the bony prominences and tendon attachments of the joint and is constructed in the form of a shaped member (21) of a soft or soft-resilient material, wherein at least one friction core (30) of a hard or incompressible material is disposed and, in the material of the shaped member (21), within the region of the affected tendons, is fixated in such a way that, when a movement is executed, a massage transversally to the course of these tendons takes place.

2. Joint bandage according to Claim 1,
characterized in that
the friction core (30) possesses a hardness which is greater in comparison with the hardness of the material of the shaped member (21).

3. Joint bandage according to either Claim 1 or 2,
characterized in that
the difference between the hardness of the shaped member (21) and the hardness of the friction core (30) is at least 10 Shore A, by preference 20 Shore A.

4. Joint bandage according to any of Claims 1 to 3,
characterized in that
the material of the shaped member (21) possesses a hardness of below 50 Shore A and the material of the friction core (30) possesses a hardness of above 50 Shore A.

5. Joint bandage according to any of Claims 1 to 4,
characterized in that
the friction core (30) is constructed so as to be disc-shaped or bar-shaped and possessing a circular cross-section or in that the friction core (30) possesses some other geometric configuration.

6. Joint bandage according to any of Claims 1 to 5,
characterized in that
the disc-shaped friction core (30), for the positional fixation in the shaped member (21) of the pressure pad (20), within the region of its circumferential wall area (31), possesses a grooving-like recess, constriction, such as a groove, undercuts, denticulation or suchlike (32) for the accommodation of the shaped member material.

7. Joint bandage according to any of Claims 1 to 4,
characterized in that
the material of the friction core (30) is fused with the material of the shaped member (21) and undetachably connected with the material of the shaped member, while the friction core (30) and the shaped member (21) are comprised of plastics, more particularly silicon rubbers or suchlike possessing different degrees of hardness.

8. Joint bandage according to any of Claims 1 to 4,
characterized in that
the friction core (30) is obtained during the fabrication of the shaped member (21) by the material curing of a section of the shaped member (21), in which case the friction core (30) and the shaped member (21) is comprised of plastics, in particular silicon rubbers or suchlike possessing different degrees of hardness.

9. Joint bandage according to any of Claims 1 to 8,
characterized in that
the shaped member (21) possesses a flat base (22) and an outward curvature (23) facing the joint but facing away from the base.

10. Joint bandage according to Claim 9,
characterized in that
the outward curvature (23) of the shaped member (21) tapers conically from one end (24) to the other (25) and in that the friction core (30) is disposed within the region of the outward curvature (23) poassesing the largest cross-section.

11. Joint bandage according to any of Claims 1 to 10,
characterized in that
the shaped member (21) possesses an approximately rectangular configuration with longitudinal sides (28, 29) tapering conically toward one shaped member end (25) and having curvedly constructed narrow sides (16, 27), while the curved narrow sides (26,27) are based upon different diameters (R1, R2).

12. Joint bandage according to Claim 11,
characterized in that
the diameter (R1) of the circ le (K1) forming the basis of the curved narrow side (26) is approximately 4cm and the diameter (R2) of the circle (K2) forming the basis of the curved narrow side (27) is approximately 2cm with a length of the shaped member (21) of approximately 11cm and a height of the outward curvature (23) of approximately 1cm within the area of the narrow side (26).

13. Joint bandage according to any of Claims 1 to 12,
characterized in that
the shaped member (21) is comprised of felt, cellular rubber, neoprene, rubber, a viscoelastic silicon rubber or of an elastic, compressible, compressively deformable silicon rubber or of a material possessing the same elasticity properties, such as natural rubber, foamed silicon or the like.

14. Joint bandage according to any of Claims 1 to 12,
characterized in that
the shaped member (21) is constructed in a pouch-like fashion and is provided with a filling of a gaseous or liquid medium, while the friction core (30) is positionally fixated on the inner wall area of the pouch.

15. Joint bandage according to any of Claims 1 to 14,
characterized in that
friction core (30) is comprised of an incompressible plastic, such as natural or synthetic rubber, hard rubber, chloroprene polymerizate, a rubber-like crosslinked polyurethane, polyamide, metal, wood or suchlike.

16. Joint bandage according to any of Claims 1 to 16,
characterized in that,
the bandage (10), on two oppositely located sides, carries pressure pads (20) with friction cores (30).

17. Joint bandage according to any of Claims 1 to 16,
characterized in that,
in the shaped member (21) of the pressure pad (20), a recess (40) is provided accommodating the friction core (30).

18. Joint bandage according to Claim 17,
characterized in that
the friction core (30) is detachably retained in the recess (40) by means of a press fit or force fit.

19. Joint bandage according to any of Claims 1 to 18,
characterized in that
the upper wall area of the friction core (30) is constructed so as to be curved, semicircular or flat with rounded corners.

## Revendications

1. Bandage articulaire en tissu élastique pour bandage, en particulier en forme de tuyau ou de coiffe, pour le rétablissement plus rapide de la fonction d'articulations lésées, telles que les articulations du genou, de l'astragale, de l'épaule, du coude et/ou de la main, avec au moins une garniture configurée comme une pelotte de pression qui, à l'état appliqué, appuie sur l'articulation, **caractérisé en ce** que la pelotte de pression (20) a une forme déterminée par les saillies osseuses et les naissances des tendons de l'articulation et qui est formée comme un corps moulé (21) en une matière souple ou souple et élastique dans lequel au moins un noyau de friction (30) en une matière dure ou incompressible est placé et qui est fixé dans la matière du corps moulé (21) dans la zone des naissances des tendons malades de telle manière qu'il y a massage, lors d'un mouvement, transversalement par rapport au sens des fibres de ces tendons.

2. Bandage articulaire selon la revendication 1, **caractérisé en ce** que le noyau de friction (30) a une dureté supérieure à celle de la matière du corps moulé (21).

3. Bandage articulaire selon l'une des revendications 1 et 2, **caractérisé en ce** que la différence entre la dureté du corps moulé (21) et la dureté du noyau de friction (30) est d'au moins 10 Shore A, et de préférence de 20 Shore A.

4. Bandage articulaire selon l'une des revendications 1 à 3, **caractérisé en ce** que la matière du corps moulé (21) a une dureté inférieure à 50 Shore A et que la matière du noyau de friction (30) a une dureté supérieure à 50 Shore A.

5. Bandage articulaire selon l'une des revendications 1 à 4, **caractérisé en ce** que le noyau de friction (30) est configuré en forme de disque ou de barre avec une section circulaire ou que le noyau de friction (30) a une autre forme géométrique.

6. Bandage articulaire selon l'une des revendications 1 à 5, **caractérisé en ce** que le noyau de friction en forme de disque (30) présente, pour fixer la position dans le corps moulé (21) de la pelotte de pression (20) dans la zone de sa surface de paroi périphérique (32) un évidement, un retrait en forme de rainure, comme une gorge, une contre-dépouille, une denture ou équivalent (32) pour loger la matière du corps moulé.

7. Bandage articulaire selon l'une des revendications 1 à 4, **caractérisé en ce** que la matière du noyau de friction (30) est fondue avec la matière du corps moulé (21) et est reliée de manière indétachable à la matière du corps moulé, le noyau de friction (30) et le corps moulé (21) étant constitués par des matières synthétiques, en particulier par de caoutchoucs au silicone ou équivalent.

8. Bandage articulaire selon l'une des revendications 1 à 4, **caractérisé en ce** que le noyau de friction (30) est obtenu, lors de la fabrication du corps moulé (21), par durcissement de la matière d'une section du corps moulé (21), le noyau de friction (30) et le corps moulé (21) étant constitués par des matières synthétiques, en particulier par des caoutchoucs au silicone ou équivalent de différents degrés de dureté.

9. Bandage articulaire selon l'une des revendications 1 à 8, **caractérisé en ce** que le corps moulé (21) présente une surface de base (22) plate et un bombement extérieur (23) tourné vers l'articulation et détourné de la surface de base.

10. Bandage articulaire selon la revendication 9, **caractérisé en ce** que le bombement extérieur (23) du corps moulé (21) s'effile d'une extrémité (24) à l'autre (25) et que le noyau de friction (30) est placé dans la zone du bombement extérieur (23) qui présente la plus grande section.

11. Bandage articulaire selon l'une des revendications 1 à 10, **caractérisé en ce** que le corps moulé (21) a une forme à peu prés rectangulaire avec des grands côtés (28, 29) qui s'effilent en une extrémité de corps moulé et avec des petits côtés (26, 27) configurés de forme arquée, différents diamètres de cercle (R1, R2) étant à la base des petits côtés arqués (26, 27).

12. Bandage articulaire selon la revendication 11, **caractérisé en ce** que le diamètre de cercle (R1) du cercle (K1) qui est à la base du petit côté arqué (26) est d'à peu près 4 cm et que le diamètre de cercle (R2) du cercle (K2) qui est à la base du petit côté arqué (27) est d'à peu près 2 cm pour une longueur du corps moulé (21) d'à peu près 11 cm et une hauteur du bombement extérieur (23) d'à peu près 1 cm dans la zone du petit côté (26).

13. Bandage articulaire selon l'une des revendications 1 à 12, **caractérisé en ce** que le corps moulé (21) est en feutre, en caoutchouc spongieux, en néoprène, en caoutchouc, en un caoutchouc au silicone visco-élastique ou en un caoutchouc au silicone élastique, compressible, déformable par pression ou une matière avec les mêmes propriétés élastiques comme le caoutchouc naturel, la mousse de silicone ou équivalent.

14. Bandage articulaire selon l'une des revendications 1 à 12, **caractérisé en ce** que le corps moulé (21) est configuré en forme de sac et a un remplissage en milieu gazeux ou liquide, le corps de friction (30) étant fixé dans sa position à la surface de paroi intérieure du sac.

15. Bandage articulaire selon l'une des revendications 1 à 14, **caractérisé en ce** que le corps de friction (30) est constitué par une matière synthétique incompressible comme un caoutchouc naturel ou synthétique, du caoutchouc dur, un polymère au chloroprène, un polyuréthane réticulé élastique comme du caoutchouc, un polyamide, du métal, du bois ou équivalent.

16. Bandage articulaire selon l'une des revendications 1 à 15, **caractérisé en ce** que le bandage (10) porte des pelottes de pression (20) avec des noyaux de friction (30) sur deux côtés opposés l'un à l'autre.

17. Bandage articulaire selon l'une des revendications 1 à 16, **caractérisé en ce** qu'un évidement (40), qui loge le noyau de friction (30), est formé dans le corps moulé (21).

18. Bandage articulaire selon la revendication 17, **caractérisé en ce** que le noyau de friction (30) est maintenu détachable dans l'évidement (40) par coïncement ou par ajustage serré.

19. Bandage articulaire selon l'une des revendications 1 à 18, **caractérisé en ce** que la surface de paroi supérieure du noyau de friction (30) est configurée en forme d'arc, de demi-cercle ou est plate avec des coins arrondis.
